# EUROPEAN PATENT SPECIFICATION

(11) **EP 2 867 322 B1**
(45) Date of publication and mention of the grant of the patent: **06.01.2021**
(21) Application number: 13722269.1
(22) Date of filing: 08.05.2013
(51) Int. Cl.: C09J 177/12, C09J 7/35

(54) **BIODEGRADABLE LINERLESS ADHESIVE TAPES AND LABELS**
BIOLOGISCH ABBAUBARE KLEBEBÄNDER UND ETIKETTEN OHNE TRENNSCHICHT
RUBANS ET ÉTIQUETTES ADHÉSIFS BIODÉGRADABLES ET SANS DOUBLURE

(30) Priority: 28.06.2012 US 201261665342 P
(43) Date of publication of application: 06.05.2015
(73) Proprietor: Dow Global Technologies LLC, Midland, MI 48674 (US)
(72) Inventor: KOOPMANS, Rudolf J., CH-8840 Einsiedeln (CH)
(74) Representative: Boult Wade Tennant LLP
(86) International application number: PCT/US2013/040103
(87) International publication number: WO 2014/003895

(56) References cited:
- WO-A1-02/36702
- WO-A1-2007/030791
- WO-A2-03/020803

## Description

### REFERENCE TO RELATED APPLICATIONS

The present application claims the benefit of U.S. Provisional Application No. 61/665,342, filed on June 28, 2012.

### BACKGROUND

### Field of the Invention

The present invention relates to the field of adhesive labels and tapes. More particularly, it relates to the field of biodegradable, linerless labels and tapes.

### Background of the Art

Adhesive labels and tapes are typically made up of substrates that are coated with pressure sensitive adhesives (PSAs). In order for the PSA to not stick to a backing paper or substrate prior to desired placement and application, a liner, or "backing paper," may be used. This separable liner is usually relatively easy to remove and is eventually wasted. Thus, this method and means is inherently environmentally unfriendly. Other means have been explored by researchers to avoid the problem of backing paper waste.

For example, WO20081124 describes laminate structures including, in one potential embodiment, a polyester amide as an adhesive layer and also a polyolefin layer.

US Patent 5,700,344 describes use of a two-part adhesive including 10 to 50 weight percet (wt%) of biodegradable thermoplastic resin having a molecular weight (Mn) greater than 30,000 grams per mole; and 20 to 90 wt% of a biodegradable tackifying resin comprising a polylactic acid composition having a number average molecular weight (Mn) of less than 20,000 grams per mole (g/mol) and a glass transition temperature (T_{g}) of less than 60°C.

US Patent 7,125,824 describes improved thermally imaged markings for use on linerless label stock comprising a substrate and a coating that is chromogenic.

WO 2007/030791 A1 describes the use of polyester amide copolymers as hot melt adhesives in laminate structures wherein the hot melt adhesive is itself a layer of the structure. The formulation includes both plasticizers and tackifiers.

US Patent 6,172,167 discloses specific polyester-amide polymers having good mechanical and environmental properties to make pressure sensitive adhesives. The polymers consist of building blocks with the general structure -(CB-VB)- wherein CB represents a block of constant length and VB represents a block of variable length. When the number average molecular weight is greater than 10,000 grams per mole, the polymer compositions have increased strength, stiffness, elasticity and ductility properties and display improved film and fiber forming properties.

WO 02/36702 relates to biodegradable tear-off strips, which can be used in conjunction with biodegradable packaging materials as biodegradable packaging.

WO 03/020803 relates to an adhesive film, which is substantially biodegradable and a layer with biodegradable adhesive is applied to at least one surface, preferably one surface only, of said film.

While these and other inventions may offer means of adhering labels and tapes to substrates, they do not solve the problem of providing biodegradable, linerless labels or tapes that are relatively or substantially tack-free (as determined by, for example, PSTC No. 5 or FINAT No. 9 or similar ASTM protocols) and/or exhibit improved peel test results (for example, ASTM D3300/D3300M) and/or shear test results (for example, ASTM D3654/D3654M) at room and/or typical storage temperatures, and potentially up to from 60°C to 150°C, enabling simplified storage and placement on the desired substrate, but which may then be easily heated to affix to the substrate.

### SUMMARY OF THE INVENTION

In one aspect the invention provides a linerless label or tape comprising a substantially biodegradable, woven or non-woven, natural or synthetic substrate and, impregnated therein, a formulation comprising at least 90 weight percent of a polyester amide segmented block copolymer.

In another aspect the invention provides a process for preparing a linerless label or tape comprising impregnating a substantially biodegradable, woven or non-woven, natural or synthetic substrate with a formulation comprising at least 90 weight percent of a polyester amide segmented block copolymer.

In yet another aspect the invention provides a process for applying a label or tape to a substrate comprising placing a surface of a linerless label or tape comprising a substantially biodegradable, woven or non-woven, natural or synthetic substrate, the substrate having been impregnated with a formulation comprising at least 90 weight percent of a polyester amide segmented block copolymer; on or against a surface of a second substrate; and applying thereto heat at a temperature greater than the melt temperature of the polyester amide segmented block copolymer; under conditions such that the surface of the linerless label or tape is affixed to the surface of the second substrate.

### DETAILED DESCRIPTION OF THE EMBODIMENTS

The invention provides linerless labels and tapes that are convenient to store and place, due to their potentially non-tacky properties at room temperature and up to the melting point of the selected polyester amide composition, but which can be easily affixed to a desired substrate using a moderately increased temperature, preferably ranging from 60°C to 150°C. The labels may also be printed, and are linerless and substantially biodegradable, thus reducing their environmental impact significantly. While the industry generally defines "linerless labels" as being PSA labels that are self-wound and have a release coating on the backside of the facestock such that the finished product does not stick to other labels on the roll, the term "linerless" as used herein means simply that the inventive labels do not require a separable additional layer to prevent them from sticking to any other surface prior to affixing them to a desired surface, the separable additional layer then being wasted. Also in contrast with the industry use of the term, the inventive linerless labels and tapes do not require any kind of release coating in order to facilitate unwinding.

The labels and tapes themselves may be in woven or non-woven form; may be synthetic or natural (e.g., biobased) in constitution; and may be cellulose based, for example, paper, cardboard or cotton; polyolefin based, such as polyethylene or polypropylene; polyamide based, such as nylon, silk or wool; polyester based, such as polylactic acid (PLA); or a combination thereof. By "biobased" is meant that such may include materials that involve synthetic modification of natural materials (e.g., PLA), materials prepared via natural microbiological activity (e.g., hydroxybutyrate), and the like. It is necessary that such label or tape be fully or substantially biodegradable. By "substantially" is meant generally that such may be at least 90 percent by weight degraded to environmentally friendly compounds by subjection to natural conditions, including for example exposure to weather, composting conditions, and combinations thereof, within a given time period.

The invention employs as an impregnant a thermoplastic polyester-amide block copolymer or formulation including this thermoplastic polyester-amide block copolymer. In one embodiment this copolymer is selected from the group consisting of:
(a) a polymer comprising repeat units -[H1-AA]- and -[DV-AA]-, where H1 is -R-CO-NH-Ra-NH-CO-R-O- or -R-NH-CO-R-CO-NH-R-O- where Ra is R or a bond, R is independently in each occurrence an aliphatic or heteroaliphatic, alicyclic or heteroalicyclic or aromatic or heteroaromatic group, AA is a -CO-R'-CO-O- where R' is a bond or an aliphatic group, where DV is -[R"-O]- and R" is an aliphatic or heteroaliphatic, alicyclic or heteroalicyclic or aromatic or heteroaromatic group;
(b) a polymer comprising repeat units -[H1-AA]-, -[DV-AA]-, and -[D2-O-AA]-, where D2 is independently in each occurrence an aliphatic or heteroaliphatic, alicyclic or heteroalicyclic or aromatic or heteroaromatic group;
(c) a polymer comprising repeat units -[H1-AA]-, -[R-O-AA]-, and -M-(AA)ₙ-, wherein M is an n valent organic moiety, and n is 3 or more;
(d) a polymer comprising repeat units -[H1-AA]-, -[R-O-AA]-, and -PA-(CO-O-R)ₙ-, wherein PA is an n valent organic moiety, and n is 3 or more;
(e) a polymer comprising repeat units -[H2-D]-, -[R-O-AA]-, and -M-(AA)ₙ-, where H2 is-CO-R-CO-NH-R-NH-CO-R-CO-O- where R is independently in each occurrence an aliphatic or heteroaliphatic, alicyclic or heteroalicyclic or aromatic or heteroaromatic group, and where D is -[R-O]-;
(f) a polymer comprising repeat units -[H2-AA]-, -[R-O-AA]-, and -PA-(COOR)ₙ-;
(g) a polymer having the formula HO-D1-O-[-CO-AA1,2-CO-O-D1-O-]ₓ-[CO-AA1,2-CO-O-AD-O]_{y}-H, wherein O-D1-O represents the residual of the diol functionality, wherein CO-AA1,2-CO represents the residual of the aliphatic dicarboxylic acid functionality or the high boiling point diacid ester functionality, wherein O-AD-O represents the residual of the polyamid diol functionality, wherein x and y are the number of repeat units in the polymer block inside the brackets;
(h) a polymer comprising repeat units -[H2-D]-, -[H2-O-D2]-, [D-AA]-, and -[D2-O-AA]-;
(i) a polymer having the formula HO-D1-O-[-CO-AA1,2-CO-O-D1-O-]ₓ-[CO-AA1,2-CO-O-CO-DD-CO]_{y}-OH, wherein O-CO-DD-CO-O represents residual of the diamide diacid functionality; and
(j) mixtures thereof.

In a more specific embodiment, the thermoplastic polyester-amide block copolymer may be selected from the group consisting of:
(a) a polymer comprising repeat units -[H1-AA]- and -[DV-AA]-, where H1 is -R-CO-NH-Ra-NH-CO-R-O- or -R-NH-CO-R-CO-NH-R-O- where Ra is R or a bond, R is independently in each occurrence an aliphatic or heteroaliphatic, alicyclic or heteroalicyclic or aromatic or heteroaromatic group, AA is a -CO-R'-CO-O- where R' is a bond or an aliphatic group, where DV is -[R"-O]- and R" is an aliphatic or heteroaliphatic, alicyclic or heteroalicyclic or aromatic or heteroaromatic group;
(b) a polymer comprising repeat units -[H1-AA]-, -[DV-AA]-, and -[D2-O-AA]-, where D2 is independently in each occurrence an aliphatic or heteroaliphatic, alicyclic or heteroalicyclic or aromatic or heteroaromatic group;
(c) a polymer comprising repeat units -[H1-AA]-, -[R-O-AA]-, and -M-(AA)ₙ-, wherein M is an n valent organic moiety, and n is 3 or more;
(d) a polymer comprising repeat units -[H1-AA]-, -[R-O-AA]-, and -PA-(CO-O-R)ₙ-, wherein PA is an n valent organic moiety, and n is 3 or more;
(e) a polymer comprising repeat units -[H2-D]-, -[R-O-AA]-, and -M-(AA)ₙ-, where H2 is-CO-R-CO-NH-R-NH-CO-R-CO-O- where R is independently in each occurrence an aliphatic or heteroaliphatic, alicyclic or heteroalicyclic or aromatic or heteroaromatic group, and where D is -[R-O]-;
(f) a polymer comprising repeat units -[H2-AA]-, -[R-O-AA]-, and -PA-(COOR)ₙ-;
(g) a polymer having the formula HO-D1-O-[-CO-AA1,2-CO-O-D1-O-]ₓ-[CO-AA1,2-CO-O-AD-O]_{y}-H, wherein O-D1-O represents the residual of the diol functionality,
   wherein CO-AA1,2-CO represents the residual of the aliphatic dicarboxylic acid functionality or the high boiling point diacid ester functionality, wherein O-AD-O represents the residual of the polyamid diol functionality, wherein x and y are the number of repeat units in the polymer block inside the brackets;
(h) a polymer comprising repeat units -[H2-D]-, -[H2-O-D2]-, [D-AA]-, and -[D2-O-AA]-;
(i) a polymer having the formula HO-D1-O-[-CO-AA1,2-CO-O-D1-O-]ₓ-[CO-AA1,2-CO-O-CO-DD-CO]_{y}-OH, wherein O-CO-DD-CO-O represents residual of the diamide diacid functionality; and
(j) mixtures thereof.

In another embodiment, the invention comprises a polymer including a first repeat unit represented by the formula -[H1-AA]- and a second repeat unit represented by the formula -[DV-AA]- in a hot melt adhesive formulation, where H1 is -R-CO-NH-Ra-NH-CO-R-O- or-R-NH-CO-R-CO-NH-R-O- where Ra is R or a bond, R is independently in each occurrence an aliphatic or heteroaliphatic, alicyclic or heteroalicyclic or aromatic or heteroaromatic group, preferably R is an aliphatic group of 1 to 10, preferably 1-4 carbon atoms and AA is a -CO-R'-CO-O- where R' is a bond or an aliphatic group, preferably of 1 to 10, preferably 2-4 carbon atoms, where DV is -[R"-O]- and R" is an aliphatic or heteroaliphatic, alicyclic or heteroalicyclic or aromatic or heteroaromatic group. Preferably, R" is selected such that R" (OH)₂ can be distilled off from the reaction mixture in subsequent derivation of the polymer. Preferably, R" is an aliphatic group of 1 to 8, more preferably 2 to 4, carbon atoms. The molecular weight of the polymer is preferably less than 2000 grams per mole.

In the preceding embodiment, the polymer may be represented as having the formula HOD1-O-[-CO-AA1-CO-O-D1-O-]x-[CO-AA1-CO-O-AD-O]_{y}-H, wherein O-D1-O represents the residual of a volatile diol functionality, wherein CO-AA1-CO represents the residual of an aliphatic dicarboxylic acid functionality (preferably short e.g. 6 or fewer carbon atoms), and O-AD-O represents a residual of a preferably short (e.g. preferably 6 or fewer carbon atoms in the diamine) symmetrical, crystallizing amide diol functionality, wherein x and y are the number of each repeat units preferably selected such that the number average molecular weight of the polymer is less than 2,000 grams per mole. It should be noted that, while for convenience the repeat units are as shown above, the polymer is not necessarily an AB block copolymer. Rather, the polymer will preferably have segments with an average of 2 repeat units of the same type per segment. Order of addition and time of addition of monomers will impact blockiness of the structure.

In another embodiment, the copolymer comprises repeat units -[H1-AA]-, -[DV-AA]-, and - [D2-O-AA]-, where D2 is independently in each occurrence an aliphatic or heteroaliphatic, alicyclic or heteroalicyclic or aromatic or heteroaromatic group, and preferably D2 is an aliphatic group. Thus, according to one representation the polymer of this embodiment may be represented as having the formula HO-D2-O-[-CO-AA1-CO-O-D1,2-O-]ₓ-[CO-AA1-CO-O-AD-O]_{y}-H, wherein O-D2-O represents a residual non-volatile diol functionality, wherein CO-AA1-CO represents the residual of the aliphatic dicarboxylic acid functionality, wherein O-AD-O represents the residual of the polyamid diol functionality, wherein O-D1,2-O represents the residual of the volatile diol functionality or the nonvolatile diol functionality, wherein x and y are the number of each of the repeat units in the polymer. Nonvolatile diols are defined in this specification as having a molecular weight greater than 1,7 heptane diol. As noted previously, while for convenience the repeat units are as shown above, the polymer is not necessarily an AB block copolymer. Rather, the polymer will preferably have segments with an average of 2 repeat units of the same type per segment. Order of addition and time of addition of monomers will impact blockiness of the structure. The number average molecular weight of the transformed polymer preferably being greater than 4,000 grams per mole.

In another embodiment, the invention includes a copolymer comprising repeat units -[H1-AA]-, -[R-O-AA]-, and -M-(AA)ₙ- in a hot melt adhesive formulation, wherein M is an n valent organic moiety, preferably aliphatic or heteroaliphatic, alicyclic or heteroalicyclic or aromatic or heteroaromatic group, preferably having up to 20 carbon atoms, and n is 3 or more. Thus, according to one representation (with a single polyfunctional moiety M built in the chain, though a plurality of M is possible) the copolymer of this embodiment may have the formula HO-D1-O-[-CO-AA1-CO-O-D1-O-]ₓ-[CO-AA1-CO-O-AD-O]_{y}-CO-AA1-CO-O-M-(O-[CO-AA1-CO-O-D1]ₓ-O-[CO-AA1-CO-O-AD-O]_{y}-H)ₙ₋₁, wherein O-D1-O represents the residual of the diol functionality, wherein CO-AA1-CO represents the residual of the aliphatic dicarboxylic acid functionality, wherein O-AD-O represents the residual of the polyamid diol functionality, wherein x and y are the number of each of the repeat units in the polymer, the number average molecular weight of the polymer preferably being greater than 4,000 grams per mole.

In yet another embodiment, the invention uses a copolymer comprising repeat units -[H1-AA]-, -[R-O-AA]-, and -PA-(CO-O-R)ₙ- in a hot melt adhesive formulation, wherein PA is an n valent organic moiety, preferably aliphatic or heteroaliphatic, alicyclic or heteroalicyclic or aromatic or heteroaromatic group, preferably having up to 20 carbon atoms, and n is 3 or more. Thus, according to one representation (with a single polyfunctional moiety PA built in the chain, though a plurality of PA is possible) the polymer may have the formula HO-D1-O-[-CO-AA1-CO-O-D1-O-]ₓ-[CO-AA1-CO-O-AD-O]_{y}-CO-PA-(CO-O-D1-[O-OC-AA1-CO-O-D1-O]ₓ-[CO-AA1-CO-O-AD-O]_{y}-H)ₙ₋₁, wherein O-D1-O represents the residual of the diol functionality, wherein CO-AA1-CO represents the residual of the aliphatic dicarboxylic acid functionality, wherein O-AD-O represents the residual of the polyamid diol functionality, wherein x and y are the number of each of the repeat units in the polymer, the number average molecular weight of the polymer preferably being greater than 4,000 grams per mole. Note that while for convenience the repeat units are as shown above, the polymer is not necessarily a block copolymer. Rather the polymer will preferably have segments with an average of 2 repeat units of the same type per segments. Order of addition and time of addition of monomers will impact blockiness of the structure.

In another embodiment, the invention includes a copolymer comprising repeat units -[H2-D]-, -[R-O-AA]-, and -M-(AA)ₙ- in a hot melt adhesive formulation, where H2 is -CO-R-CO-NH-R-NH-CO-R-CO-O- where R is independently in each occurrence an aliphatic or heteroaliphatic, alicyclic or heteroalicyclic or aromatic or heteroaromatic group, preferably R is an aliphatic group of 1 to 10, preferably 2-4 carbon atoms and where D is -[R-O]- and R is a an aliphatic or heteroaliphatic, alicyclic or heteroalicyclic or aromatic or heteroaromatic group. According to one representation, the polymer of this embodiment may be represented by the formula (with a single polyfunctional moiety M built in the chain, though a plurality of M is possible): HO-D1-O-[-CO-AA1-CO-O-D1-O-]ₓ-[O-D1-O-CO-DD-CO-]_{y}-O-M-(O-[CO-AA1-CO-O-D1]ₓ-O-[O-D1-O-CO-DD-CO]_{y}-OH)ₙ₋₁, wherein O-D1-O represents the residual of the diol functionality, wherein CO-AA1-CO represents residual of the aliphatic dicarboxylic acid functionality, wherein O-CO-DD-CO-O represents residual of the diamide diacid functionality, wherein x and y are the number of each repeat units in the polymer. Preferably, the polymer has a number average molecular weight greater than 4000. Note that while for convenience the repeat units are shown as the above structure the polymer is not necessarily a strict block copolymer. Rather the polymer will preferably have segments with an average of 2 repeat units of the same type per segments. Order of addition and time of addition of monomers will impact blockiness of the structure.

In still another embodiment, the invention uses a copolymer comprising repeat units -[H2-AA]-, -[R-O-AA]-, and -PA-(COOR)n- in a hot melt adhesive formulation. According to one representation of this embodiment (with a single polyfunctional moiety PA built in the chain, though a plurality of PA is possible) the polymer may be represented by the formula HO-D1-O-[-CO-AA1-CO-O-D1-O-]ₓ-[OC-DD-CO-O-D1-O]_{y}OC-PA-([-CO-O-D1-O-CO-AA1-CO-]ₓ[O-D1-O-CO-DD-CO]_{y}-OH)ₙ₋₁, wherein O-D1-O represents the residual of the diol functionality, wherein CO-AA1-CO represents residual of the aliphatic dicarboxylic acid functionality, wherein O-CO-DD-CO-O represents residual of the diamide diacid functionality, wherein x and y are the number of each of the repeat units in the polymer. Preferably, the polymer has a number average molecular weight greater than 4000. As previously noted, while for convenience the repeat units are shown as the above structure the polymer is not necessarily a strict block copolymer. Rather, the polymer will preferably have segments with an average of 2 repeat units of the same type per segment. Order of addition and time of addition of monomers will impact blockiness of the structure.

In another embodiment, the invention includes a copolymer having the formula HO-D1-O-[-CO-AA1,2-CO-O-D1-O-]ₓ-[CO-AA1,2-CO-O-AD-O]_{y}-H, wherein O-D1-O represents the residual of the diol functionality, wherein CO-AA1,2-CO represents the residual of the aliphatic dicarboxylic acid functionality or the high boiling point diacid ester functionality, wherein O-AD-O represents the residual of the polyamid diol functionality, wherein x and y are the number of repeat units in the polymer block inside the brackets. The number average molecular weight of the polymer is preferably greater than 4,000 grams per mole. Once more, it is noted that, while for convenience the repeat units are as shown above, the polymer is not necessarily an AB block copolymer. Rather, the polymer will preferably have segments with an average of 2 repeat units of the same type per segment. Order of addition and time of addition of monomers will impact blockiness of the structure.

In still another embodiment, the invention includes a copolymer comprising repeat units-[H2-D]-, -[H2-O-D2]-, [D-AA]- (preferably, -[DV-AA]-), and -[D2-O-AA]- in a hot melt adhesive formulation. Thus, according to one embodiment the transformed polymer may be represented by the formula HO-D2-O-[-CO-AA1-CO-O-D1,2-O-]ₓ-[O-D1,2-O-CO-DD-CO]_{y}-OH, wherein O-D2-O represents the residual of the nonvolatile diol functionality, wherein CO-AA1-CO represents the residual of the aliphatic dicarboxylic acid functionality, wherein O-CO-DD-CO-O represents the residual of the diamide diacid functionality, wherein O-D1,2-O represents the residual of the volatile diol functionality or the nonvolatile diol functionality, wherein x and y are the number of each of the repeat units in the polymer, the number average molecular weight of the polymer is preferably greater than 4,000 grams per mole. It should again be noted that, while for convenience the repeat units are as shown above, the polymer is not necessarily a strict block copolymer. Rather, the polymer will preferably have segments with an average of 2 repeat units of the same type per segments. Order of addition and time of addition of monomers will impact blockiness of the structure.

In yet another embodiment, the invention may use a polymer having the formula HO-D1-O-[-CO-AA1,2-CO-O-D1-O-]ₓ-[CO-AA1,2-CO-O-CO-DD-CO]_{y}-OH, wherein O-D1-O represents the residual of the diol functionality, wherein CO-AA1,2-CO represents residual of the aliphatic dicarboxylic acid functionality or the high boiling point diacid ester functionality, wherein O-CO-DD-CO-O represents residual of the diamide diacid functionality, wherein x and y are the number of repeat units in the polymer block inside the brackets. The number average molecular weight of the polymer is preferably greater than 4,000 grams per mole. Note that while for convenience the repeat units are shown as the above structure the polymer is not necessarily a strict block copolymer. Rather the polymer will preferably have segments with an average of 2 repeat units of the same type per segments. Order of addition and time of addition of monomers will impact blockiness of the structure.

It should be noted that in the formulas shown in this application the oxygen in the repeat unit or portion of repeat units are drawn as occurring on one end of the repeat unit or portion of the repeat unit. However, the oxygen could have been shown on the other end of the repeat unit or portion of the repeat unit and still represented the same actual structure. Therefore, the structures as drawn herein shall be recognized as representing both variants.

A particular and distinct advantage of the invention is that, unlike previous use of similar polymers in hot melt adhesive formulations, the invention does not require use of a tackifier, plasticizer, or combination thereof. Elimination of these components, and in fact, the need to use essentially no other components in the invention, ensures that the tapes or labels will remain tack-free at temperatures including room temperature and up to generally about 60°C, while still ensuring convenient application at higher temperatures. Elimination of these components also eliminates any need for liners, thereby decreasing waste. Finally, elimination of these components ensures convenient biodegradability of the impregnant, thus ensuring overall biodegradability of the tape or label where the tape or label is, in turn, formed of a biodegradable substrate such as, for example, a cellulosic material. Nonetheless, addition of tackifiers, plasticizers, fillers, natural and mineral oils, waxes, and the like may be carried out as desired. In general it is preferred that the copolymer be at least 90 wt%; more preferably at least 95 wt%, of the total impregnant formulation.

The process of the invention includes the steps of preparing the polymer and using it to impregnate the selected tape or label. Impregnation is defined herein as filling of voids or pores, and thus is contrasted with "coating" or "layering," which involves application of a material such that it covers either only or substantially only the surface of a given substrate. Impregnation involves melting the polymer at a temperature above its melting point and essentially squeezing the melted copolymer into the tape or label, continuing to squeeze to remove excess polymer, a method called "solution impregnation." The relative amount of the copolymer in comparison with the surface of the tape or label, alternatively referred to as substrate or first substrate herein, depends upon the desired concentration based upon the actual surface area of the substrate, measured in grams per square meter (g/m2). The amount of surface area therefore will depend upon the "voidage," which is a measure of the porosity of the substrate. Those skilled in the art will recognize that with routine experimentation including testing for lap shear, as described further in the Examples section hereinafter, optimization of concentration for the impregnation may be conveniently achieved.

The invention also includes a process for applying a linerless label or tape to a substrate, such as, for example, a package. In this process the impregnated, linerless tape or label is placed on or against a surface of the desired substrate; and then heat is applied to the impregnated tape or label at a temperature above the melting point of the polyester amide block copolymer; to adhere the tape or label to the substrate. For the polymers defined hereinabove those having an amide level of at least about 10 mole percent (mol%) will have a melting point of at least about 60°C, while those having an amide level of about 70 mol% will have a melting point of about 150°C. Thus, it is generally desirable that an amide level and corresponding melting point range from 10 mol% and 60°C, respectively, and 70 mol% and 150°C, respectively. The source of the heat may be a heat gun or equivalent equipment on a larger scale, such as commercial scale heating.

One advantage of the linerless tapes and labels of the invention is that they are capable of being printed by any suitable means that is capable of printing on the given substrate of which they are formed. Typical inks may be those based on, for example, urethanes, epoxides, acrylics, or acrylates, and may be applied by means such as rotational printers. This advantage is further facilitated by elimination of any need for inclusion of a pressure-sensitive adhesive (PSA) on the final tape or label.

The polymers of the invention can be prepared as described in U.S. Pat. No. 6,172,167 and/or in applicants' international application number PCT/US2006/023450. U.S. Pat. No. 6,172,167 teaches a process for producing aliphatic polyester-amide polymers having the formula HO-D1-O-[-CO-AA1-CO-O-D1-O-]ₓ-[CO-AA1-CO-O-AD-O]_{y}-H, wherein O-D1-O represents a diol functionality, wherein CO-AA1-CO represents a short (preferably 6 or fewer carbon atoms) aliphatic dicarboxylic acid functionality, wherein O-AD-O represents a short (e.g. preferably 6 or fewer carbon atoms in the diamine) symmetrical, crystallizing amid diol functionality, wherein x and y are the number of repeat units in the polymer block inside the brackets. As taught in U.S. Pat. No. 6,172,167, such polymers can be made from reaction mixtures comprising an amide diol. Amide diols which are particularly useful in the practice of the instant invention have the following structure:

HO-(CH₂)ₙ-CONH-(CH₂)ₘ-(X)ₖ-(CH₂)ₘ-CONH-(CH₁)ₙ-OH

wherein X is NH, O or S, k is from 0 to 1, m is from 1 to 4 and n is from 4 to 6..

The amide diol can be prepared by any suitable means; however, it has been found advantageous to prepare the amide diol by the ring opening polymerization (ROP) reaction between at least one primary diamine and at least one lactone. The preparation of the amide diol can also be carried out according to the methods described in U.S. Pat. No. 3,025,323 and in "Synthesis of Alternating Polyamideurethans by Reacting Diisocyanates with N,N'-Di-(6-hydroxycaproyl)alkylenediamines and N-hydroxy-alkyl-6-hydroxycaproamide" by S. Katayama et al. in J. Appl. Polym. Sci., Vol. 15, 775-796 (1971).

A primary diamine is defined in this specification as an organic compound comprising two primary amine groups. The primary diamine may also comprise secondary and tertiary amines groups. Suitable diamines are ethylene diamine, diethylene triamine, butane diamine and hexane diamine.

The lactone preferably has 4, 5 or 6 carbon atoms. Suitable lactones include γ-butyrolactone, δ-valerolactone, s-caprolactone, pentadeca lactone, glycolide and lactides.

The preferred method of carrying out such reaction is to mix, in a stainless steel stirred-tank reactor, the lactone with the diamine in a ratio of at least 2 mol of lactone per mol of diamine, preferably in a ratio of 2.0 to 2.5 mol of lactone per mol of diamine. The reaction is preferably carried out under a nitrogen blanket. The reactants may be dissolved in a solvent, but generally it is preferable to carry out the reaction in the absence of a solvent in order to eliminate the effort required in separating the solvent from the polymer composition product. Preferably the reaction temperature is maintained at a temperature which is lower than the melting point of the pure amide diol, preferably between 0 degrees Celsius (° C.) and 30° C. lower than the melting point, which generally results in a product comprising a high fraction of the desired amine diol product which can be used in subsequent process steps without the need for further purification. If the reaction is carried out in the absence of a solvent the whole contents of the reactor will generally solidify. It is generally advantageous to allow the reaction mixture cool down to ambient temperature and to allow the reaction product to stand for a several hours, preferably for more than 6 hours, more preferably for more than 12 hours to allow any remaining diamine to react. The amide diol product may then be removed from the reactor by heating the reactor contents, preferably under a suitable inert gas blanket, until the product melts.

A particularly preferred amide diol is the condensation product prepared from ethylene diamine and ε-caprolactone, coded C2C in the examples and which has the following structure:

HO-(CH₂)₅-CONH-(CH₂)₂-NHCO-(CH₂)₅-OH

The aliphatic polyester-amide polymer can be made by contacting an amide diol with a low molecular weight dicarboxylic acid diester and a low molecular weight diol, heated to liquefy the mixture after which the catalyst is injected.

Low molecular weight dicarboxylic acid diesters are defined as having a molecular weight less than 258 grams per mole. The alkyl moieties of the dicarboxylic acid diester are preferably the same or different and have between 1 and 3 carbon atoms. Preferably the alkyl moieties are methyl groups. The dicarboxylate moiety of the dicarboxylic acid diester preferably has between 2 and 8 carbon atoms, most preferably between 4 and 6 carbon atoms. Preferably the dicarboxylate moiety is a succinate, glutarate or adipate group. Suitable dicarboxylic acid esters include dimethyl succinate, dimethyl adipate, dimethyl oxalate, dimethyl malonate and dimethyl glutarate.

Generally the reaction may be carried out in a stirred heated reactor or devolitizer, fitted with a reflux column, under an inert gas blanket. In a preferred embodiment solid amide diol is first mixed with the dicarboxylic acid diester. The mixture of amide diol and dicarboxylic acid diester is then slowly heated up to a temperature of about 140° C. or until such temperature that the amide diol dissolves completely. The mixture of amide diol and dicarboxylic acid diester mixture is then maintained at this temperature for 1.5 to 3 hours. To minimize discoloration the bis-amide diol is first mixed with dimethyladipate at ambient temperature and then the mixture is heated to make it liquid and at the same time it is believed that the most reactive free amine functions are captured by transamidation reaction with dimethyladipate to amide functions. Then the diol is added and finally the catalyst (at a moment when the most aggressive species are believed to have reacted away. The low molecular weight diol is introduced in stoichiometric excess, the mixture is homogenized and finally the catalyst is injected to form the aliphatic polyester-amide pre-polymer having a number average molecular weight less than 2000 grams per mole.

Volatile diols are defined in this specification as having a molecular weight of less than 1,8-octane diol. Suitable diols include monoethylene glycol, 1,3-propanediol, 1,4-butanediol, 1,5 pentane diol, 1,6 hexane diol and 1,7 heptane diol. The volatile diol is added to the polymer and the mixture is generally homogenized by continuous stirring. The temperature is generally maintained at or above the melting temperature of the amide diol, typically at about 140° C. The reaction is preferably carried out under an inert gas blanket at about atmospheric pressure. A catalyst is then preferably added to the reactant mixture. Any suitable compound for catalyzing transesterification and transamidification reactions may be used. Suitable catalysts include tetrabutoxy titanium (IV), zinc acetate and magnesium acetate.

The addition of the volatile diol and optional catalyst results in the evolution of a vapor comprising the low molecular weight alcohol or alcohol mixture corresponding to the alkyl moiety or moieties of the dicarboxylic acid esters, and the formation of the pre-polymer. The vapor formed is distilled off at about atmospheric pressure from the reaction mixture comprising the pre-polymer. The reaction is continued until the evolution of alcohol subsides.

In a second stage of the polycondensation process the reaction is continued in a devolatizer reactor under reduced pressure to completely remove the free volatile diols and to increase the molecular weight and convert the pre-polymer with molecular weight less than 2000 gram/mole to a full polyester amide polymer with molecular weight higher than 4000 gram/mole. At this point in time other reactive species like non-volatile diols can be admixed as to further increase the molecular weight or to introduce specific properties like branching or hydrophobic interactions.

A polymer suitable for use herein can be made by contacting an aliphatic polyester-amide polymer having the formula HO-D1-O-[-CO-AA1-CO-O-D1-O-]ₓ-[CO-AA1-CO-O-AD-O]_{y}-H with a nonvolatile diol having the formula HO-D2-OH to form a mixture, the temperature of the mixture being sufficiently high to produce a transformed material comprising a transformed polymer having the formula HO-D2-O-[-CO-AA1-CO-O-D1,2-O-]ₓ-[CO-AA1-CO-O-AD-O]_{y}-H.

Another polymer suitable for use herein can be made by contacting an aliphatic polyester-amide polymer having the formula HO-D1-O-[-CO-AA1-CO-O-D1-O-]ₓ-[CO-AA1-CO-O-AD-O]_{y}-H with a polyol having the formula M-(OH)ₙ to form a mixture, wherein n is 3 or more, the temperature of the mixture being sufficiently high to produce a transformed material comprising a transformed polymer having the formula HO-D1-O-[-CO-AA1-CO-O-D1-O-]ₓ-[CO-AA1-CO-O-AD-O]_{y}-CO-AA1-CO-O-M-(O-[CO-AA1-CO-O-D1]ₓ-O-[CO-AA1-CO-O-AD-O]_{y}-H)ₙ₋₁. M in the polyol M-(OH)ₙ is an n valent organic moiety, preferably aliphatic or heteroaliphatic, alicyclic or heteroalicyclic or aromatic or heteroaromatic group, preferably having up to 20 carbon atoms. More preferably, M is aliphatic. Preferred examples of M-(OH)ₙ include glycerine, trimethylolpropane, pentaerythritol, methylglucoside, sorbitol, and ethoxylated and propoxylated derivatives of those molecules.

Another polymer suitable for use herein can be made by contacting an aliphatic polyester-amide polymer having the formula HO-D1-O-[-CO-AA1-CO-O-D1-O-]ₓ-[CO-AA1-CO-O-AD-O]_{y}-H with a polyacid ester having the formula PA-(CO-ORb)ₙ to form a mixture, wherein n is 3 or more, the temperature of the mixture being sufficiently high to produce a transformed material comprising a transformed polymer having the formula HO-D1-O-[-CO-AA1-CO-O-D1-O-]ₓ-[CO-AA1-CO-O-AD-O]_{y}-CO-PA-(CO-O-D1-[O-OC-AA1-CO-O-D1-O]ₓ-[CO-AA1-CO-O-AD-O]_{y}-H)ₙ₋₁. PA in the polyacid ester PA-(CO-ORb)ₙ is an n valent organic moiety, preferably aliphatic or heteroaliphatic, alicyclic or heteroalicyclic or aromatic or heteroaromatic group, preferably having up to 20 carbon atoms. Preferred PA include 1,3,5 benzene tricarboxylic acid; citric acid, agaric acid, and aconitic acid. Rb is an aliphatic group of 1-10 carbon atoms, preferably 1-6 carbons, more preferably -CH3, -CH2-CH3, propyl or isopropyl.

Another polymer suitable for use herein can be made by contacting an aliphatic polyester-amide polymer having the formula HO-D1-O-[-CO-AA1-CO-O-D1-O-]ₓ-[CO-AA1-CO-O-AD-O]_{y}-H with a high boiling point diacid ester having the formula RO-CO-AA2-CO-OR to form a mixture, the temperature of the mixture being sufficiently high to produce a transformed material comprising a transformed polymer having the formula HO-D1-O-[-CO-AA1,2-CO-O-D1-O-]ₓ-[CO-AA1,2-CO-O-AD-O]_{y}-H.

Another polymer suitable for use herein can be made by contacting a pre-polymer having the formula HO-D1-O-[-CO-AA1-CO-O-D1-O-]ₓ-[O-D1-O-CO-DD-CO]_{y}-OH, wherein O-D1-O represents the residual of a volatile diol functionality, wherein O-CO-DD-CO-O represents the residual of a short (e.g. preferably 6 or fewer carbon atoms) symmetrical, crystallizing diamide diacid functionality, with a nonvolatile diol having the formula HO-D2-OH to form a mixture, the temperature of the mixture being sufficiently high to produce a transformed material comprising a transformed polymer having the formula HO-D2-O-[-CO-AA1-CO-O-D1,2-O-]ₓ-[O-D1,2-O-CO-DD-CO]_{y}-OH.

Yet another polymer suitable for use herein can be made by contacting a polymer having the formula HO-D1-O-[-CO-AA1-CO-O-D1-O-]ₓ-[O-D1-O-CO]_{y}-OH with a polyol having the formula M-(OH)ₙ to form a mixture, wherein n is 3 or more, the temperature of the mixture being sufficiently high to produce a transformed material comprising a transformed polymer having the formula HO-D1-O-[-CO-AA1-CO-O-D1-O-]ₓ-[O-D1-O-CO-DD-CO-]_{y}-O-M-(O-[CO-AA1-CO-O-D1]ₓ-O-[O-D1-O-CO-DD-CO]_{y}-OH)ₙ₋₁.

Another polymer suitable for use herein can be made by contacting a polymer having the formula HO-D1-O-[-CO-AA1-CO-O-D1-O-]ₓ-[O-D1-O-CO-DD-CO]_{y}-OH with a polyacid ester having the formula PA-(CO-OR)ₙ to form a mixture, wherein n is 3 or more, the temperature of the mixture being sufficiently high to produce a transformed material comprising a transformed polymer having the formula HO-D1-O-[-CO-AA1-CO-O-D1-O-]ₓ-[OC-DD-CO-O-D1-O]_{y}OC-PA-([-CO-O-D1-O-CO-AA1-CO-]ₓ[O-D1-O-CO-DD-CO]_{y}-OH)ₙ₋₁.

Yet another polymer suitable for use herein can be made by contacting a polymer having the formula HO-D1-O-[-CO-AA1-CO-O-D1-O-]ₓ-[O-D1-O-CO-DD-CO]_{y}-OH with a high boiling point diacid ester having the formula RO-CO-AA2-CO-OR to form a mixture, the temperature of the mixture being sufficiently high to produce a transformed material comprising a transformed polymer having the formula HO-D1-O-[-CO-AA1,2-CO-O-D1-O-]ₓ-[CO-AA1,2-CO-O-CO-DD-CO]_{y}-OH.

The short symmetrical, crystallizing diamide diacid functionality herein is the same as defined and taught in the above-referenced U.S. Pat. No. 6,172,167. A particularly preferred di-amide diacid functionality is the condensation product prepared from ethylene diamine and dimethyl adipate, coded A2A in the examples.

In this specification high boiling point dicarboxylic acid diesters are defined as aliphatic dicarboxylic acid diesters having a molecular weight greater than 202. The alkyl moieties of the dicarboxylic acid diester are preferably the same or different and have between 1 and 3 carbon atoms. Preferably the alkyl moieties are methyl groups. The dicarboxylic acid moiety preferably has between 7 and 10 carbon atoms, most preferably either 9 or 10 carbon atoms. Preferably the dicarboxylic acid moiety is an azelate or sebacate group. Preferred dicarboxylic acid esters are dimethyl azelate, dimethyl sebacate and dimethyl suberate.

Suitable nonvolatile diols in the instant invention include higher glycols such as dipropylene glycol or tripropylene glycol, polyethylene glycols (PEG's of molecular weight 400 to 8000) and EO capped polypropylene glycols of molecular weight 400 to 4000), dimer diols or Soy polyols or other high molecular weight natural diols like mentioned in Jetter et al. Phytochemistry 55, 169-176 (2000). Polyols suitable for use in the instant invention include glycerol, trimethylol propane, sorbitol and sucrose.

The reaction of the aliphatic polyester-amide polymer with the nonvolatile diol, the polyol, polyacid ester or the high boiling point dicarboxylic acid diester is generally carried out under an inert gas blanket. The mixture is then heated over a period of typically 2 to 3 hours to a temperature of about 180°C. or to such temperature that the resulting amide ester polymer remains in the molten or dissolved state. The pressure is typically about atmospheric pressure. The reaction can result in the evolution of low molecular weight alcohol which is removed by distillation from the system.

The pressure in the reactor is then gradually lowered to an absolute pressure of about 5 millibar to initiate the distillation under vacuum of any remaining volatile materials. The resulting polymer composition can then be cooled to about 150°C. and brought to atmospheric pressure, after which the polymer may be removed from the reactor whilst still in the molten state.

The viscosity of the copolymer or copolymer impregnant formulation is preferably less than 100 Pa·sec. at 190°C. More preferably, the viscosity of the hot melt adhesive of the instant invention is in the range of from 5 to 50 Pa·sec. at 160°C. Preferably, the glass transition temperature of the hot melt adhesive of the instant invention is less than 20°C. Preferably, the melting point of the hot melt adhesive of the instant invention is higher than 60°C. Preferably, the hot melt adhesive of the instant invention exhibits a Newtonian viscosity as rheological property. The product is resistant to cold water but is removed from the substrate by application of hot water or steam.

The tapes and labels of the present invention are also surmised to be repulpable.

As used herein, the term "aliphatic" refers to hydrocarbons which are saturated or unsaturated (alkanes, alkenes, alkynes) and which may be straight-chain or branched. Aliphatic groups can be optionally substituted with various substituents or functional groups, including among others halides, hydroxy groups, thiol groups, ester groups, ketone groups, carboxylic acid groups, amines, and amides. A "heteroaliphatic" group is an aliphatic group that contains one or more non-carbon atoms in the hydrocarbon chain (e.g., one or more non-neighboring CH₂ groups are replaced with O, S or NH).

The term "alicyclic" refers to hydrocarbons that have one or more saturated or unsaturated rings (e.g., three to ten-membered rings) and which may be bicyclic. Alicyclic groups can include portions that are branched and/or straight-chain aliphatic in combination with cyclic hydrocarbon. Alicyclic groups can be substituted, as noted above for aliphatic groups. A "heteroalicyclic" group is an alicyclic group that contains one or more heteroatoms (non-carbon atoms) in the hydrocarbon chain, in a ring or in a straight-chain or branched aliphatic portion of the alicyclic group (e.g., one or more non-neighboring CH₂ groups can be replaced with O, S or NH).

The term "aromatic" refers to hydrocarbons that comprise one or more aromatic rings which may be fused rings (e.g., as in a naphthalene group). Aromatic groups can include portions that are branched and/or straight-chain aliphatic and/or alicyclic in combination with aromatic. Aromatic groups can be substituted, as noted above for aliphatic groups. A "heteroaromatic" group is an aromatic group that contains one or more heteroatoms (non-carbon atoms) in an aromatic ring (e.g., a pyridine ring). A CH in an aromatic ring can be replaced with O, S or N. In any alicyclic or aliphatic portion of an aromatic groups, one or more non-neighboring CH₂ groups can be replaced with a heteroatom (e.g., O, S, NH).

While the instant invention has been described above according to its preferred embodiments, it can be modified within the scope of this disclosure. This application is therefore intended to cover any variations, uses, or adaptations of the instant invention using the general principles disclosed herein. Further, the instant application is intended to cover such departures from the present disclosure as come within the known or customary practice in the art to which this invention pertains and which fall within the limits of the following claims.

### EXAMPLES

### Preparation of the Amide Diol Ethylene-N,N"-Dihydroxyhexanamide (C2C)

C2C monomer is prepared by reacting 1.2 kg ethylene diamine (EDA) with 4.56 kg of s-caprolactone under a nitrogen blanket in a stainless steel reactor equipped with an agitator and a cooling water jacket. An exothermic condensation reaction between the ε-caprolactone and the EDA occurs which causes the temperature to rise gradually to 80°C. A white deposit forms and the reactor contents solidify, at which the stirring is stopped. The reactor contents are then cooled to 20°C. and are then allowed to rest for 15 hours. The reactor contents are then heated to 140°C at which temperature the solidified reactor contents melt. The liquid product is then discharged from the reactor into a collecting tray. A nuclear magnetic resonance study of the resulting product shows that the molar concentration of C2C in the product exceeds 80 percent. The melting point of the C2C product is determined to be 140°C.

Contacting C2C with Dimethyl Adipate.

A devolitizer reactor is charged with 2.622 kg liquid dimethyl adipate and 2.163 kg of the solid C2C diamide diol produced as described above. The reactor contents are brought slowly under nitrogen purge to a temperature of 140°C. in order to melt the C2C in the reaction mixture.

Contacting the Composition with 1,4-Butanediol without Further Addition of Non Volatile Diols, Acids or Branching Agents.

1.352 kg of 1,4-butandiol are added to the reactor contents followed by 105 milliliters (mL) of a 10 percent by weight solution of tetrabutoxy titanium (IV) in 1,4-butanediol. The resulting reaction results in the formation of methanol which is then removed as a vapor by the nitrogen purge from the reactor system. The pressure in the system is maintained at atmospheric pressure, and temperature is gradually raised to 180°C. The reaction and distillation of methanol is continued until the evolution of methanol subsides. The pressure in the reactor is then lowered to an absolute pressure of 450 mbar and then stepwise to 20 mbar, resulting in further evolution of methanol vapor from the reaction mixture. When the flow of methanol subsides the pressure in the reactor is further lowered an absolute pressure of 0.25 mbar to initiate distillation of 1,4-butandiol, and the temperature in the reactor is gradually increased to 200°C When 710 mL of 1,4-butanediol has been recovered from the reactor, the vacuum in the reactor is broken and the resulting molten amide ester polymer composition is discharged from the reactor.

The above procedure is repeated to prepare six different batches of amide ester block copolymer compositions at 50 mole % C2C content calculated on the total amount of diols incorporated in the polymer structure (Samples 1-6, respectively) having the following physical properties.

**Table 1**

| Sample | Molecular Weight Based on NMR | Melt Temp (°C) | Melt Viscosity (pa*s at 180°C) | Ultimate Tensile Strength (MPa) | Elongation to Break (%) | Modulus |
|---|---|---|---|---|---|---|
| 1 | 16000 | 127 | 18 | 17 | 700 | 240 |
| 2 | 19000 | 132 | 21 | 21 | 735 | 279 |
| 3 | 10800 | 124 | 18 | 17 | 840 | 290 |
| 4 | 9000 | 123 | 12 | 15 | 725 | 290 |
| 5 | 11800 | 122 | 24 | 23 | 970 | 260 |
| 6 | 8500 | 118 | 7 | 12 | 640 | 230 |

| | | | | | | |
|---|---|---|---|---|---|---|
| *Measured with Brookfield Viscometer | | | | | | |

The viscosity of the materials of the instant invention is generally Newtonian up to number average molecular weight of 20,000 grams/mole.

### Preparation of Di-Amide Di-Ester Monomer A2A

In a nitrogen atmosphere, titanium (IV) butoxide (0.92 g, 2.7 mmol), ethylene diamine (15.75 g, 0.262 mol), and dimethyl adipate (453.7 g, 2.604 mol) are loaded into a 3-neck, 1 L roundbottom flask that is stoppered and transferred to hood. Flask is placed under positive nitrogen via inlet adaptor attached to a Firestone valve. Stir-shaft with blade is inserted into flask along with stir bearing with overhead stir motor. Stoppered condenser is inserted into flask. A thermocouple inserted thru septa is also inserted into the flask. Flask is warmed with a hemisphere heating mantle that is attached to proportional temperature controller. Basic reaction profile is 2.0 hours to/at 50°C, 2.0 hours to/at 60°C, 2.0 hours to/at 80°C; overnight at 100 C. Flask is slowly cooled with stirring to ^{∼}50°C, stirring stopped and cooled to ^{∼}room temperature. Approximately 200 mL of cyclohexane is add to flask with agitation for a filterable slurry with solid collected on a medium porosity glass filtration funnel. Collected solids are washed twice with 50 mL of cyclohexane. Product is dried overnight in an ^{∼}50°C vacuum oven. Dried product is broken up and re-slurried in fresh cyclohexane (^{∼}300 mL), recollected by filtration, rinsed twice with ^{∼}50 mL cyclohexane, and dried to constant weight in a 50°C vacuum oven under full pump vacuum. Yield is 59.8 g (66%).

Contacting the A2A Monomer Composition with 1,4-Butanediol ("1,4 BD") without Further Addition of Non Volatile Diols, Acids or Branching Agents.
PBA A2A-50% (polyester amide with 50 mole % A2A monomer incorporation)

The devolatizer reactor is charged at room temperature (or 50-60°C) with 348.4 g (2.0 moles) of dimethyl adipate (DMA) followed by 680 g (^{∼}7.7 moles) 1,4 butane diol and 688.8 g (2.0 moles) of A2A (powder); with nitrogen blanket. The kneader temperature is slowly brought to 140-150°C under nitrogen purge to ensure complete solvatation (clear solution) of the contents.

Then, still under nitrogen blanket and at 140-150°C, Ti(OBu)₄ catalyst is injected as 41.5 gram of a 10% by weight solution in 1,4 BD (4000 ppm calculated on total esters; 4.15 g catalyst+37.35 g BD; total content of 1,4 BD is 717 g or 7.97 moles). At 140-150°C, methanol starts distilling. The reactor temperature is increased stepwise to 175°C at atmospheric pressure; initially with low (to prevent entrainment of the monomers DMA and BD) nitrogen sweep applied. Methanol fraction is distilled off and collected (theoretical amount: 256 g, 8 moles) in a cooling trap. The purpose is to maintain a constant stream of methanol distilled. When the major fraction of methanol is removed at 175°C, the temperature is increased to 190°C and the reactor pressure is stepwise decreased first slowly to 50-20 mbar (to avoid eventual foaming) and further to 5 mbar to complete the methanol removal and to initiate the 1,4 BD distillation. The pressure is further decreased<1 mbar, until the steady distillation of 1,4 butane diol is observed. At the end of the reaction the temperature is raised to 200-220°C. Calculated amount of 1,4 BD collected: 360 g (4 moles). When the 1,4 butane diol removal is completed, the reactor is cooled to ^{∼}150°C (depending on torque measured) and brought to atmospheric pressure under nitrogen blanket and the polymer is collected.

The following additional resins were produced according to the methods described above. The monomers C2C and A2A were incorporated at two levels each, specifically at 25 and 50 mole %. The materials are coded PEA-C2C25%, PEA-C2C50%, PEA-A2A25% and PEA-A2A50% respectively. Data are collected in the table below. From each material 2 mm thick compression molded plaques were produced. Prior to compression molding, the materials were dried at 65°C under vacuum for about 24 hours. Plaques of 160x160x2 mm were obtained by compression molding isothermally at 150°C, 6 minutes at 10 bar and afterwards 3 minutes at 150 bar. The samples were cooled from 150°C to room temperature at 20°C/min. The physical property data are presented in the following table.

**Table 2**

| | Polyester-Amide C2C 50%* | Polyester-Amide C2C 25% | Polyester-Amide A2A 50% | Polyester-Amide A2A 25% |
|---|---|---|---|---|
| Modulus (MPa) | 370 | 155 | 360 | 130-140 |
| Tensile (MPa) | 15-20 | 6 | 15 | 6-12 |
| Elongation (%) | 600-800 | 330 | 600 | 600-1200 |
| T_{crystallization} (°C) | 115(s) | 65(w) | 140(w) | 125(w) |
| Melt Viscosity at 180°C in Pa*s | 5-15 | 3-10 | 25-40 | 7-12 |

| | | | | |
|---|---|---|---|---|
| * refers to mole % amide segment. ** refers to the temperature of crystallization when cooled from the melt; crystallization in sharp (s) or wider (w) temperature range. | | | | |

### Mechanical Properties

Load-deformation response of different PEA materials was measured on an Instron 5564 load frame. The tests are done on dog bone samples which have been conditioned for one week at 23°C and 50% humidity. The modulus is determined, using an extensometer, at a crosshead speed of 1 mm/min. After modulus determination the crosshead speed is changed to 50 mm/min.

### DMS (Solid State Rheology)

The shear modulus was measured on the Advanced Rheometric Expansion System (ARES) with torsion rectangular setup.

Conditions: Dynamic Temperature Ramp tests were performed from -140°C. till 140°C at a heating rate of 2°C/min under a strain of 0.2% with a frequency of 10 rad/s. A rectangular sample with a width of about 12.6 mm and a length of about 25 mm was cut out of the compression molded plaque. Measurements were performed under nitrogen atmosphere and auto-tension option active.

### Dynamic Viscosity (Melt Rheology)

The complex viscosity was measured on the Advanced Rheometric Expansion System (ARES) with parallel plate setup.

Conditions frequency sweep: Dynamic Frequency Sweep tests were performed from 100 till 0.1 rad/sec (in logarithmic mode, 10 points per decade). The strain varied from 10% to 30% depending on the viscosity of the sample (still in the linear region) in order to obtain a reasonable torque level. A 25 mm disk was cut out of the compression molded plaque. Measurements were performed under nitrogen atmosphere.

DSC experiments were performed on a TA Instruments Q1000 instrument Conditions TA Q1000:
Purge gas: nitrogen (50 ml/min)
Standard aluminum pans; sample size: 5-7 mg.
Temperature calibration with Indium.
Temperature program: equilibrate at -80°C; -80°C. to 170°C (20°C/min); 170°C (5 min); 170°C to -80°C (20°C/min); equilibrate at -80°C; -80°C. to 170°C (20°C/min).

A linerless tape is prepared by preparing a kraft paper tape and impregnating it with the polyester-amide block copolymer described hereinabove by squeezing the copolymer into the tape at a temperature above the copolymer's melting point. Squeezing is carried out such that excess copolymer is removed and the tape is then cooled to a temperature below the melting point of the copolymer at a rate of 10°C/min, under ambient conditions and for a time period ranging from 1 to 30 seconds, to solidify the polymer. At this point the tape is non-tacky. The tape is then rolled and stored for an additional 30 days. Following this time the tape is removed from storage and, using commercial roller tape application equipment, the tape is unrolled, applied to a series of kraft paper-wrapped packages, cutting the tape between packages, and the tape is simultaneously heated using a heating iron device to a temperature above the copolymer's melting point. The impregnated paper tape adheres to the kraft paper-wrapped packages. Each impregnated surface is then allowed to cool to a temperature below the copolymer's melting point and an ink imprint is applied to the tape as desired.

## Claims

1. A linerless label or tape comprising a substantially biodegradable, woven or non-woven, natural or synthetic substrate and, impregnated therein, a formulation comprising at least 90 weight percent of a polyester amide segmented block copolymer.

2. The linerless label or tape of claim 1 wherein the substrate is selected from the group consisting of materials based on cellulose, a polyolefin, a polyamide, a polyester, and combinations thereof.

3. The linerless label or tape of any of claims 1 to 2 wherein the formulation further comprises up to 10 weight percent of an additive selected from fillers, natural and mineral oils, tackifiers, waxes, and combinations thereof.

4. A process for preparing a linerless label or tape comprising impregnating a substantially biodegradable, woven or non-woven, natural or synthetic substrate with a formulation comprising at least 90 weight percent of a polyester amide segmented block copolymer.

5. The process of claim 4 wherein the substrate is selected from the group consisting of materials based on cellulose, a polyolefin, a polyamide, a polyester, and combinations thereof.

6. The process of claim 4 or 5 wherein the formulation further comprises up to 10 weight percent of an additive selected from fillers, natural and mineral oils, tackifiers, waxes, and combinations thereof.

7. A process for applying a label or tape to a substrate comprising placing a surface of a linerless label or tape comprising a substantially biodegradable, woven or non-woven, natural or synthetic substrate, the substrate having been impregnated with a formulation comprising at least 90 weight percent of a polyester amide segmented block copolymer; on or against a surface of a second substrate; and applying thereto heat at a temperature greater than the melt temperature of the polyester amide segmented block copolymer; under conditions such that the surface of the linerless label or tape is affixed to the surface of the second substrate.

8. The process of claim 7 wherein the first substrate is selected from the group consisting of materials based on cellulose, a polyolefin, a polyamide, a polyester, and combinations thereof.

9. The process of claim 7 or 8 wherein the formulation further comprises up to 10 weight percent of an additive selected from fillers, natural and mineral oils, tackifiers, waxes, and combinations thereof.

## Patentansprüche

1. Ein trägerbandloses Etikett oder Band, das Folgendes beinhaltet: ein im Wesentlichen biologisch abbaubares, natürliches oder synthetisches Gewebe- oder Vliessubstrat und, darin imprägniert, eine Zubereitung, die zu mindestens 90 Gewichtsprozent ein segmentiertes Polyesteramid-Blockcopolymer beinhaltet.

2. Trägerbandloses Etikett oder Band gemäß Anspruch 1, wobei das Substrat ausgewählt ist aus der Gruppe, bestehend aus Materialien auf Basis von Cellulose, einem Polyolefin, einem Polyamid, einem Polyester und Kombinationen davon.

3. Trägerbandloses Etikett oder Band gemäß einem der Ansprüche 1 bis 2, wobei die Zubereitung ferner bis zu 10 Gewichtsprozent ein Additiv beinhaltet, ausgewählt aus Füllstoffen, Natur- und Mineralölen, Klebrigmachern, Wachsen und Kombinationen davon.

4. Ein Verfahren zum Herstellen eines trägerbandlosen Etiketts oder Bands, das Folgendes beinhaltet: Imprägnieren eines im Wesentlichen biologisch abbaubaren, natürlichen oder synthetischen Gewebe- oder Vliessubstrats mit einer Zubereitung, die zu mindestens 90 Gewichtsprozent ein segmentiertes Polyesteramid-Blockcopolymer beinhaltet.

5. Verfahren gemäß Anspruch 4, wobei das Substrat ausgewählt ist aus der Gruppe, bestehend aus Materialien auf Basis von Cellulose, einem Polyolefin, einem Polyamid, einem Polyester und Kombinationen davon.

6. Verfahren gemäß Anspruch 4 oder 5, wobei die Zubereitung ferner zu bis zu 10 Gewichtsprozent ein Additiv beinhaltet, ausgewählt ist aus Füllstoffen, Natur- und Mineralölen, Klebrigmachern, Wachsen und Kombinationen davon.

7. Ein Verfahren zum Aufbringen eines Etiketts oder Bands auf ein Substrat, das Folgendes beinhaltet: Platzieren einer Oberfläche eines trägerbandlosen Etiketts oder Bands, das ein im Wesentlichen biologisch abbaubares, natürliches oder synthetisches Gewebe- oder Vliessubstrat beinhaltet, wobei das Substrat mit einer Zubereitung, die zu mindestens 90 Gewichtsprozent ein segmentiertes Polyesteramid-Blockcopolymer beinhaltet, imprägniert worden ist; auf oder gegen eine Oberfläche eines zweiten Substrats; und Anwenden von Wärme mit einer Temperatur höher als die Schmelztemperatur des segmentierten Polyesteramid-Blockcopolymers darauf; unter Bedingungen derart, dass die Oberfläche des trägerbandlosen Etikett oder Bands an der Oberfläche des zweiten Substrats befestigt wird.

8. Verfahren gemäß Anspruch 7, wobei das erste Substrat ausgewählt ist aus der Gruppe, bestehend aus Materialien auf Basis von Cellulose, einem Polyolefin, einem Polyamid, einem Polyester und Kombinationen davon.

9. Verfahren gemäß Anspruch 7 oder 8, wobei die Zubereitung ferner zu bis zu 10 Gewichtsprozent ein Additiv beinhaltet, ausgewählt aus Füllstoffen, Natur- und Mineralölen, Klebrigmachern, Wachsen und Kombinationen davon.

## Revendications

1. Une étiquette ou un ruban adhésif sans doublure comprenant un substrat naturel ou synthétique, tissé ou non-tissé, sensiblement biodégradable et, imprégnée dans celui-ci, une formulation comprenant au moins 90 pour cent en poids d'un copolymère bloc segmenté polyester-amide.

2. L'étiquette ou le ruban adhésif sans doublure de la revendication 1 dans lesquels le substrat est sélectionné dans le groupe consistant en des matériaux à base de cellulose, une polyoléfine, un polyamide, un polyester, et des combinaisons de ceux-ci.

3. L'étiquette ou le ruban adhésif sans doublure de n'importe lesquelles des revendications 1 à 2 dans lesquels la formulation comprend en sus jusqu'à 10 pour cent en poids d'un additif sélectionné parmi des charges, des huiles minérales et naturelles, des agents poisseux, des cires, et des combinaisons de ceux-ci.

4. Un procédé pour la préparation d'une étiquette ou d'un ruban adhésif sans doublure comprenant le fait d'imprégner un substrat naturel ou synthétique, tissé ou non-tissé, sensiblement biodégradable d'une formulation comprenant au moins 90 pour cent en poids d'un copolymère bloc segmenté polyester-amide.

5. Le procédé de la revendication 4 dans lequel le substrat est sélectionné dans le groupe consistant en des matériaux à base de cellulose, une polyoléfine, un polyamide, un polyester, et des combinaisons de ceux-ci.

6. Le procédé de la revendication 4 ou de la revendication 5 dans lequel la formulation comprend en sus jusqu'à 10 pour cent en poids d'un additif sélectionné parmi des charges, des huiles minérales et naturelles, des agents poisseux, des cires, et des combinaisons de ceux-ci.

7. Un procédé pour l'application d'une étiquette ou d'un ruban adhésif sur un substrat comprenant le fait de placer une surface d'une étiquette ou d'un ruban adhésif sans doublure comprenant un substrat naturel ou synthétique, tissé ou non-tissé, sensiblement biodégradable, le substrat ayant été imprégné d'une formulation comprenant au moins 90 pour cent en poids d'un copolymère bloc segmenté polyester-amide, sur ou contre une surface d'un deuxième substrat ; et le fait d'appliquer sur celles-ci de la chaleur à une température supérieure à la température de fusion du copolymère bloc segmenté polyester-amide, dans des conditions telles que la surface de l'étiquette ou du ruban adhésif sans doublure est fixée à la surface du deuxième substrat.

8. Le procédé de la revendication 7 dans lequel le premier substrat est sélectionné dans le groupe consistant en des matériaux à base de cellulose, une polyoléfine, un polyamide, un polyester, et des combinaisons de ceux-ci.

9. Le procédé de la revendication 7 ou de la revendication 8 dans lequel la formulation comprend en sus jusqu'à 10 pour cent en poids d'un additif sélectionné parmi des charges, des huiles minérales et naturelles, des agents poisseux, des cires et des combinaisons de ceux-ci.
